(19) Europäisches Patentamt
European Patent Office
Office européen des brevets

(11) Veröffentlichungsnummer: **0 191 058**
**B1**

(12) **EUROPÄISCHE PATENTSCHRIFT**

(45) Veröffentlichungstag der Patentschrift: **29.03.89**

(51) Int. Cl.⁴: **C 07 C 103/50,** A 61 K 31/165

(21) Anmeldenummer: **85903971.1**

(22) Anmeldetag: **31.07.85**

(86) Internationale Anmeldenummer:
**PCT/AT 85/00019**

(87) Internationale Veröffentlichungsnummer:
**WO 86/00892 (13.02.86 Gazette 86/04)**

# (54) IMMUNWIRKSAME VERBINDUNGEN.

(30) Priorität: **01.08.84 AT 2468/84**

(43) Veröffentlichungstag der Anmeldung:
**20.08.86 Patentblatt 86/34**

(45) Bekanntmachung des Hinweises auf die Patenterteilung:
**29.03.89 Patentblatt 89/13**

(84) Benannte Vertragsstaaten:
**AT BE CH DE FR GB IT LI LU NL SE**

(56) Entgegenhaltungen:
**DE-A-2 702 137**
**FR-A-2 321 881**
**US-A-3 859 315**

**Burger's medicinal chemistry, 4th edition, part III (1981), page 1264**

**Die Akte enthält technische Angaben, die nach dem Eingang der Anmeldung eingereicht wurden und die nicht in dieser Patentschrift enthalten sind.**

(73) Patentinhaber: **CL PHARMA AKTIENGESELLSCHAFT, St. Peter- Strasse 25, A-4021 Linz (AT)**

(72) Erfinder: **CL PHARMA AKTIENGESELLSCHAFT, St. Peter- Strasse 25, A-4021 Linz (AT)**

(74) Vertreter: **Kunz, Ekkehard, Dr., Chemie Holding AG Patentwesen St. Peter- Strasse 25, A-4021 Linz (AT)**

EP 0 191 058 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft die Verwendung von 2,6-bis(Monoalkylamino-acetylamino)-anthrachinonen oder von deren physiologisch unbedenklichen Säureadditionssalzen zur Herstellung immunsuppressiver Arzneimittel.

In der AT-Patentschrift 351 521 wurden bis-Acetamido-anthrachinone, insbesondere bis-Dimethylaminoacetyl-amino-anthrachinon und bis-Hexamethyleniminoacetyl-amino-anthrachinon, die zur Behandlung von Viruserkrankungen geeignet sind, geoffenbart.

Überraschend und unerwartet stellten sich die Verbindungen 2,6-bis(Monomethylamino-acetylamino)-anthrachinon und 2,6-bis(Monoethylamino-acetylamino)-anthrachinon als stark immunsuppressive Stoffe heraus.

Gegenstand der Erfindung ist daher die Verwendung von Verbindungen der allgemeinen Formel (I)

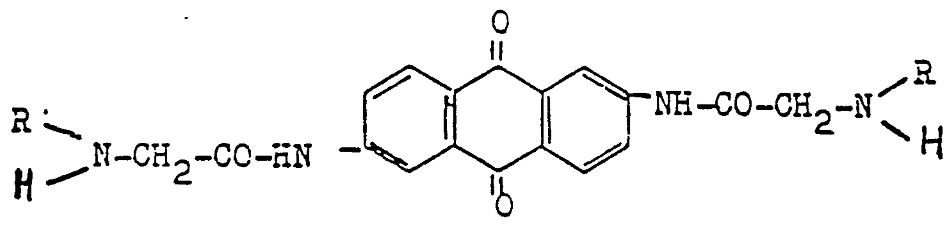

(I)

in der R Methyl oder Ethyl bedeutet oder von deren physiologisch unbedenklichen Säureadditionssalzen zur Herstellung eines immunsuppressiven Arzneimittels.

Die Herstellung der Verbindungen der allgemeinen Formel I erfolgt nach Abderhalden, Handbuch der biologischen Arbeitsmethoden, Abt. I, Teil 2, 1, Seite 979 ff., 1927 durch Umsetzung eines 2,6-Diaminoanthrachinons der Formel (II)

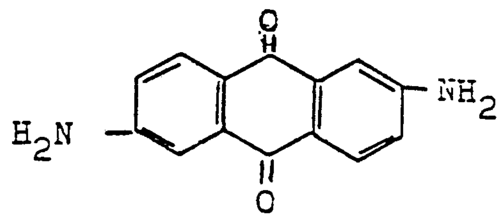

(II)

mit alpha-Chloressigsäurechlorid, worauf das so erhaltene bis-alpha-Halogenacylaminoanthrachinon der Formel (III)

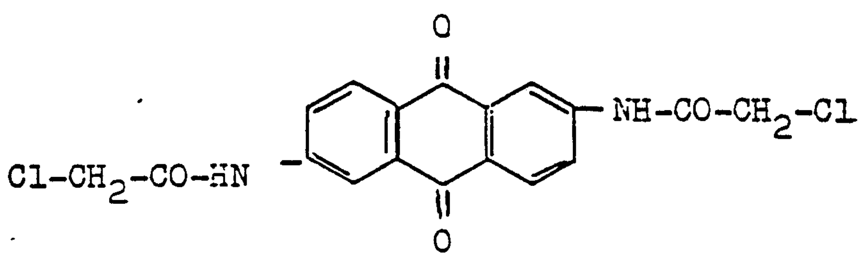

(III)

mit einer Verbindung der Formel (IV)

$R\text{-}NH_2$ IV

worin R die obgenannte Bedeutung hat, zu einer Verbindung der allgemeinen Formel I umgesetzt wird. Die so erhaltenen Anthrachinone der allgemeinen Formel I werden gegebenenfalls durch Zugabe einer physiologisch verträglichen Säure in das entsprechende Salz übergeführt.

Die Wirkstoffe oder ihre physiologisch verträglichen Säureadditionssalze liegen in pharmazeutischen Zubereitungen vor. Die für therapeutische Zwecke bestimmten Zubereitungen inhibieren die Abstoßungsreaktion von homologen Transplantaten bei Säugern, wenn sie in Mengen von 0,1 bis 50 mg/kg Körpergewicht und Tag gegeben werden. Dieser Dosierungsbereich kann der Erzielung einer optimalen therapeutischen Wirkung angepaßt werden; so können beispielsweise mehrere unterteilte Dosen verabreicht werden, oder die Dosis kann entsprechend der therapeutischen Situation vermindert werden. Die Wirkstoffe können auf jede beliebige Art und Weise verabreicht werden, z. B. oral, intraperitoneal, subkutan, intramuskulär oder intravenös. Die Wirkstoffe können allein oder in Kombination mit den üblichen pharmazeutischen Trägerstoffen zur Anwendung gelangen. Geeignete Anwendungsformen sind z. B. Tabletten, Kapseln, Zäpfchen, Lösungen, Säfte, usw.

**Herstellung der Wirkstoffe**

2,6-bis(Methylamino)-acetylamino-anthrachinon

In einem Druckgefäß wurden 3,0 g 2,6-bis-(Chloracetylamino)-anthrachinon in 150 ml Ethanol suspendiert und mit einem Überschuß an Methylamin (ca. 30 %-ige Lösung in Ethanol) versetzt. Das Gemisch wurde 3 h auf 80°C erhitzt und über Nacht bei Raumtemperatur gerührt. Nach Einengen im Vakuum wurde der ausgeschiedene Niederschlag abgesaugt und aus Ethanol umkristallisiert.

Ausbeute: 3,3 g; Fp: 263°C

Auf ähnliche Art und Weise konnte unter Verwendung der entsprechenden Ausgangsstoffe 2,6-bis(Ethylamino)-acetylamino-anthrachinon hergestellt werden.

**Biologische Wirksamkeit**

Die Wirkstoffe wurden im DTH-Test (delayed type hypersensitivity in mice) gemäß Dietrich, F. M. & Hess, R.: Hypersensitivity in mice. I. Induction of contact sensitivity to oxazolone and inhibition by various chemical compounds. Int. Arch. Allergy 38, 246 - 259 (1970) untersucht. Die in der nachstehenden Tabelle angeführten Daten zeigen die immunsuppressive Wirksamkeit von 2,6-bis-/(Methylamino)-acetylamino/-anthrachinon im Vergleich zu Cyclosporin-A (CS-A)

| Verbindung | Dosis (mg/kg) | % Hemmung |
|---|---|---|
| 2,6-bis-MAA | 1,0 | 35 |
| CS-A | 70,0 | 35 |

Die Verbindungen (2,6-bis-MAA = 2,6-bis/(Methyl-amino)-acetylamino/-anthrachinon), CS-A = Cyclosporin-A, wurden in vivo an OF 1 - Mäusen durch lokale Sensibilisierung mit Antigen (ethanolische Oxazolone-Lösung) getestet. Die Verminderung der Entzündungsreaktion gegenüber unbehandelten Tieren ist in % Hemmung angegeben.

**Patentansprüche**

1. Verwendung von Verbindungen der allgemeinen Formel (I)

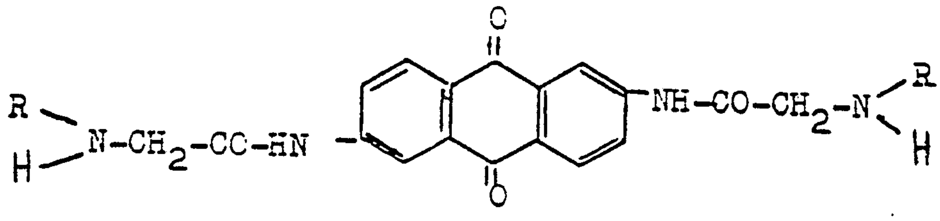

(I)

in der R Methyl oder Ethyl bedeutet oder von deren physiologisch unbedenklichen Säureadditionssalzen, zur Herstellung eines immunsuppressiven Arzneimittels.

2. Verwendung von 2,6-bis((Methylamino)-acetylamino)-anthrachinon oder von dessen physiologisch unbedenklichen Säureadditionssalzen, zur Herstellung eines immunsuppressiven Arzneimittels.

3. Verwendung von 2,6-bis((Ethylamino)-acetylamino)-anthrachinon oder von dessen physiologisch unbedenklichen Säureadditionssalzen, zur Herstellung eines immunsuppressiven Arzneimittels.

**Claims**

1. Use of compounds of the general formula (I)

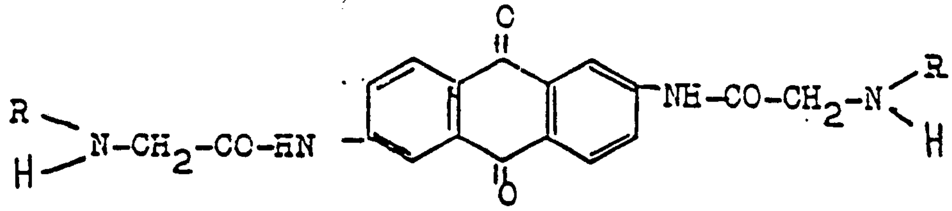

(I)

in which R denotes methyl or ethyl, or of the physiologically acceptable acid addition salts thereof, for the preparation of an immunosuppressive medicament.

2. Use of 2,6-bis(methylaminoacetylamino)anthraquinone, or of the physiologically acceptable acid addition salts thereof, for the preparation of an immunosuppressive medicament.

3. Use of 2,6-bis(ethylaminoacetylamino)anthraquinone, or of the physiologically acceptable acid addition salts thereof, for the preparation of an immunosuppressive medicament.

## Revendications

1. Utilisation de composés répondant à la formule générale (I)

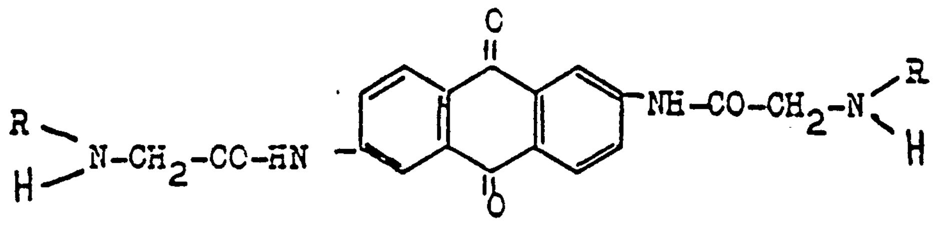

(I)

dans laquelle R représente un groupe méthyle ou éthyle, ou de leurs sels d'addition d'acides sans inconvénients physiologiques, pour la préparation d'un médicament immunosuppresseur.

2. Utilisation de la 2,6-bis((méthylamino)-acétylamino)anthraquinone ou de ses sels d'addition d'acides sans inconvénients physiologiques pour la préparation d'un médicament immunosuppresseur.

3. Utilisation de la 2,6-bis((éthylamino)acétylamino)anthraquinone ou de ses sels d'addition d'acides sans inconvénients physiologiques pour la préparation d'un médicament immunosuppresseur.